(19) Europäisches Patentamt
European Patent Office
Office européen des brevets

(11) Publication number: 0 029 460
B1

(12) EUROPEAN PATENT SPECIFICATION

(45) Date of publication of patent specification: 29.12.82

(51) Int. Cl.[3]: C 07 C 125/06

(21) Application number: 80901044.0

(22) Date of filing: 06.06.80

(86) International application number:
PCT/JP80/00126

(87) International publication number:
WO 80/02686 11.12.80 Gazette 80/28

# (54) PROCESS FOR PREPARING AROMATIC URETHANE.

(30) Priority: 06.06.79 JP 70154/79

(43) Date of publication of application:
03.06.81 Bulletin 81/22

(45) Publication of the grant of the patent:
29.12.82 Bulletin 82/52

(84) Designated Contracting States:
FR

(56) References cited:
JP - A - 51 098 240
US - A - 3 467 694

(73) Proprietor: MITSUI TOATSU CHEMICALS, INCORPORATED
2-5 3-chome Kasumigaseki
Chiyoda-Ku Tokyo 100 (JP)

(72) Inventor: MIYATA, Katsuharu
1071-2, Nakano-cho Totsuka-ku
Yokohama-shi Kanagawa 247 (JP)
Inventor: HASEGAWA, Seiji
1541, Yabe-cho Totsuka-ku
Yokohama-shi, Kanagawa 244 (JP)

(74) Representative: Lavoix, Jean et al,
c/o Cabinet Lavoix 2, Place D'Estienne D'Orves
F-75441 Paris Cedex 09 (FR)

EP 0 029 460 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Process for preparing aromatic urethane

*Field of the Invention*

This invention relates to an improved process for the preparation of aromatic urethane compounds by reacting an aromatic nitro compound, an organic compound having at least one hydroxyl group (hereinafter referred to as a hydroxyl-containing compound), and carbon monoxide at high temperature and pressure in the presence of a catalyst.

*Description of the Prior Art*

Conventionally, urethanes have been mainly prepared from isocyanates and hydroxyl-containing compounds. In recent years, novel processes for the preparation of urethanes have been developed in view of the shortage of raw materials for the manufacture of isocyanates, the steep rise in their prices, the strong toxicity of intermediate products, and the like. However, these newly developed processes have a number of serious disadvantages, which have precluded them from being put into practice on an industrial scale.

For example, there has been proposed a process for preparing urethanes from an alcohol, carbon monoxide and an aromatic nitro compound in the presence of rhodium chlorocarbonyl as a catalyst (Japanese Patent Publication No. 1420/67). In this process, however, the yield of the desired product is low even if the catalyst is used in large quantities and the reaction is carried out for a long period of time. Accordingly, it cannot be regarded as an economical process for preparing urethanes of high purity.

As a modification of the process of Japanese Patent Publication No. 1420/67, there has been proposed a process using a carbonyl-containing compound of a metal belonging to group VIII of the periodic table and a salt (e.g., ferric chloride) of a metal existing in two or more valence states (Japanese Patent Publication No. 23939/68). In this process, however, the yield of the product is low even when a mononitro compound is used as a raw material and it is still lower when a dinitro compound is used as a raw material.

In U.S. Patent 3,531,512, there has been proposed a process using a catalyst composed of palladium and a Lewis acid. Even when a dinitro compound is used as a raw material, this process can produce urethanes in a fairly good yield of 80 to 90%. However, severe conditions including an initial pressure of carbon monoxide of 190 to 350 kg/cm$^2$ and a temperature of 190° to 200°C are required for the attainment of such a high yield.

Furthermore, in the process disclosed in Japanese Patent Laid-open No. 98240/76, a catalyst system composed of a platinum group metal, a Lewis acid and a tertiary amine is used. Thus, aromatic urethanes can be obtained under mild conditions and in high yield. However, the platinum group metal used in this process is expensive. From an industrial point of view, therefore, it is desirable to reduce the amount of the platinum group metal to a minimum.

*Disclosure of the Invention*

The present inventors have made repeated studies of a process for the preparation of urethanes by reacting an aromatic nitro compound, a hydroxyl-containing compound and carbon monoxide in the presence of a catalyst system composed of (a) a platinum group metal and/or a compound thereof, (b) a Lewis acid and (c) a tertiary amine, and have discovered that a high yield of aromatic urethanes can be obtained without the above-described disadvantages of prior art processes and with a short reaction time by allowing ferrous hydroxide and/or ferric hydroxide to coexist in the reaction system. The present invention has been perfected on the basis of this discovery.

According to the process of the present invention, the reaction is accelerated so greatly that it is completed in a short period of time, the amount of platinum group metal catalyst used can be saved, and the desired aromatic urethanes can be obtained in high yield.

*Best Mode of Practising the Invention*

The aromatic nitro compound which is used as the main raw material in the process of the present invention is an aromatic compound having at least one nitro group as a nuclear substituent, and this aromatic compound may have either one nitro group of two or more nitro groups. That is, the aromatic compound may be either a mononitro compound or a polynitro compound. The aromatic compound may have other substituents in addition to the nitro group or groups.

The useful nitro compounds include, for example, substituted and unsubstituted nitrobenzenes, dinitrobenzenes, dinitrotoluenes, nitronaphthalenes, nitroanthracenes, nitrobiphenyls, bis(nitrophenyl)-alkanes, bis(nitrophenyl)ethers, bis(nitrophenyl)thioethers, bis(nitrophenyl)sulfones, nitrodiphenoxy-alkanes, heterocyclic aromatic compounds such as nitrophenothiazines and 5-nitropyrimidine, and the like. Specific examples thereof are nitrobenzene, *o*-, *m*- and *p*-nitrotoluenes, *o*-nitro-*p*-xylene, 1-nitro-naphthalene, *m*- and *p*-dinitrobenzenes, 2,4- and 2,6-dinitrotoluenes, dinitromesitylene, 4,4′-dinitrobiphenyl, 2,4-dinitrobiphenyl, 4,4′-dinitrodibenzyl, bis(4-nitrophenyl)methane, bis(4-nitrophenyl)ether, bis(2,4-dinitrophenyl)ether, bis(4-nitrophenyl)thioether, bis(4-nitrophenyl)sulfone, bis(4-

nitrophenoxy)ethane, α,α'-dinitro-*p*-xylene, α,α'-dinitro-*m*-xylene, 2,4,6-trinitrotoluene, *o*-, *m*- and *p*-chloronitrobenzenes, 2,3-, 3,4- and 2,5-dichloronitrobenzenes, 1-chloro-2,4-dinitrobenzene, 1-bromo-4-nitrobenzene, 1-fluoro-2,4-dinitrobenzene, *o*-, *m*- and *p*-nitrophenyl carbamates, *o*-, *m*- and *p*-nitroanisoles, 2,4-dinitrophenetole, ethyl *p*-nitrobenzoate, *m*-nitrobenzenesulfonyl chloride, 3-nitrophthalic anhydride, 3,3'-dimethyl-4,4'-dinitrobiphenyl, 1,5-dinitronaphthalene and the like. Isomers and mixtures of these aromatic nitro compounds, as well as homologues thereof, can also be used. In addition, aromatic nitroso compounds, azo compounds and azoxy compounds can also be used similarly to the aromatic nitro compounds.

Among the foregoing aromatic nitro compounds, mononitro compounds such as nitrobenzene, *o*-, *m*- and *p*-chloronitrobenzenes, 2,3- and 3,4-dichloronitrobenzenes, etc. and aromatic dinitro compounds such as *m*- and *p*-dinitrobenzenes, 2,4- and 2,6-dinitro toluenes, 1,5-dinitronaphthalene, etc. are particularly preferred. This is because these nitro compounds facilitate the reaction and give a high yield of the end products which have many uses as raw materials for the manufacture of drugs, agricultural chemicals, polyurethanes and the like.

The hydroxyl-containing compounds which can be used in the process of the present invention include monohydric or polyhydric alcohols containing containing a primary, secondary or tertiary hydroxyl group or groups, and monohydric or polyhydric phenols. Typical examples thereof are ethanol and phenol. The useful alcohols can be represented by the formula

$$R(OH)_n$$

where R is a straight-chain or branched alkyl, cycloalkyl, alkylene, cycloalkylene or aralkyl group and n is a whole number of 1 or more. These alcohols may also have one or more substituents containing an oxygen, nitrogen, halogen, or sulfur atom or atoms, such as halogen atoms, sulfoxide group, sulfone group, carbonyl group, carboxylate group, etc.

Specific examples of the alcohols represented by the formula $R(OH)_n$ include monohydric alcohols such as methyl alcohol, ethyl alcohol, *n*-propyl alcohol, isopropyl alcohol, *n*-butyl alcohol, isobutyl alcohol, *tert*-butyl alcohol, straight-chain or branched amyl alcohol, hexyl alcohol, cyclohexyl alcohol, lauryl alcohol, cetyl alcohol, benzyl alcohol, chlorobenzyl alcohol, methoxybenzyl alcohol, etc.; dihyric alcohols such as ethylene glycol, diethylene glycol, propylene glycol, dipropylene glycol, etc.; trihydric alcohols such as glycerol, hexanetriol, etc.; and higher polyols.

The useful phenols include phenol, chlorophenol, cresol, ethylphenol, straight-chain or branched propyl-, butyl- and higher alkylphenols, catechol, resorcinol, 4,4'-dihydroxydiphenylmethane, 2,2'-isopropylidenediphenol, anthranol, phenanthrol, pyrogallol, phloroglucinol and the like.

Among the foregoing hydroxyl-containing compounds, methanol, ethanol and isobutanol are particularly preferred. This is because these alcohols give a higher reaction rate and a higher yield of the end product than other hydroxyl-containing compounds.

The amounts of aromatic nitro compound, hydroxyl-containing compound and carbon monoxide used in the process of the present invention are desirably such that the hydroxyl group of the hydroxyl-containing compound and the carbon monoxide are present in amounts equimolar with the nitro group of the aromatic nitro compound.

The main catalyst used in the process of the present invention comprises a platinum group metal and/or a compound thereof. The useful platinum group metals and compounds thereof include elemental palladium, rhodium and ruthenium as well as compounds thereof, and they may be used alone or in admixture.

Specific examples thereof are elemental palladium, rhodium and ruthenium; halides, cyanides, thiocyanides, isocyanides, oxides, sulfates, nitrates and carbonyl compounds thereof; addition compounds or complexes thereof with tertiary amines such as triethylamine, pyridine, isoquinoline, etc.; complexes thereof with organic phosphorus compounds such as triphenylphosphine, etc.; and the like. These substances may be used either directly for purposes of reaction or after being supported on a carrier such as alumina, silica, carbon, barium sulfate, calcium sulfate, asbestos, bentonite, diatomaceous earth, fuller's earth, organic ion exchange resin, inorganic ion exchange resin, magnesium silicate, aluminum silicate, molecular sieve, etc. Alternatively, this carrier may be charged into a reactor separately from the main catalyst comprising palladium, rhodium ruthenium or a compound thereof.

Among the foregoing substances, palladium and its compounds are particularly preferred as the main catalyst. Specific examples thereof are metallic palladium, palladium chloride, palladium bromide, and palladium supported on carbon or alumina.

The amount of platinum group metal used may vary widely according to the type of platinum group metal used and other reaction conditions. However, the platinum group metal is generally used in such an amount that the weight ratio of platinum group metal to nitro compound is in the range of 1 to $1 \times 10^{-5}$ and preferably $5 \times 10^{-1}$ to $1 \times 10^{-4}$.

In the process of the present invention, a Lewis acid and a tertiary amine are used as co-catalysts. The term "Lewis acid" as used herein denotes those compounds which are described, for example, in

Jack Hine, "Physical Organic Chemistry" (McGraw-Hill Book Co., New York, 1962), and is intended to include Brønsted acids.

The useful Lewis acids include, for example, halides, oxyhalides, sulfates, acetates, phosphates, nitrates and the like of metals such as tin, titanium, germanium, aluminum, iron, copper, vanadium, nickel, zinc, cobalt, manganese and the like. Specific examples thereof are ferric chloride, ferrous chloride, stannic chloride, stannous chloride, aluminum chloride, cupric chloride, cuprous chloride, copper acetate and the like. Among these Lewis acids, ferrous chloride and ferric chloride are particularly preferred.

The amount of Lewis acid used as a co-catalyst is such that the weight ratio of Lewis acid to nitro group is in the range of 2 to $2 \times 10^{-3}$ and preferably 1 to $5 \times 10^{-2}$.

The useful tertiary amines are aliphatic, aliphatic-aromatic, alicyclic, aromatic and heterocyclic aromatic amines. These tertiary amines may either be in the unsubstituted form or have one or more suitable substituents inert to the reaction of the present invention, such as halogen atoms, alkyl groups, aryl groups, alkenyl groups, cyano group, aldehyde group, alkoxy groups, phenoxy groups, thioalkoxy groups, thiophenoxy groups, carbamyl group, carboalkoxy groups, thiocarbamyl group, etc. They include, for example, aliphatic tertiary amines such as trimethylamine, triethylamine, tripropylamine, tributylamine, etc.; aliphatic-aromatic amines such as N,N-dimethylaniline, N,N-diethylamine, N,N-diisopropylaniline, etc.; alicyclic tertiary amines such as N,N-dimethylcyclohexylamine, N,N-diethylcyclohexylamine, N,N-diisopropylcyclohexylamine, 1,4-diazabicyclo [2.2.2] octane, etc.; aromatic tertiary amines such as triphenylamine, etc.; and nitrogen-containing heterocyclic aromatic compounds such as pyridine, quinoline, isoquinoline, etc. Among these tertiary amines, nitrogen-containing heterocyclic aromatic compounds bring about a remarkable effect in enhancing the yield of the desired urethane and the reaction rate. Further specific examples of the useful nitrogen-containing heterocyclic aromatic compounds are 1-methylpyrrole, 1-phenylpyrrole, 1-methylimidiazole, 1-methylindole, 1-phenylindole, indolenine, 2-isobenzazole, indolizine, 1-methylcarbazole, 2-chloropyridine, 2-bromopyridine, 2-fluoropyridine, 4-phenylpyridine, 2-, 3- and 4-methylpyridines, 2-methyl-5-ethylpyridine, 2,6-dimethylpyridine, 2,4,6-trimethylpyridine, 2-vinylpyridine, 2-styrylpyridine, 3-chloropyridine, 2,6-dichloropyridine, 2-chloro-4-methylpyridine, 4-phenylthiopyridine, 2-methoxypyridine, 4-dimethylaminopyridine, $\alpha$-picolinic acid phenyl ester, $\gamma$-picolinic acid methyl ester, 2,6-dicyanopyridine, $\alpha$-picolinic aldehyde, $\alpha$-picolinic acid amide, 5,6,7,8-tetrahydroquinoline, 2,2-dipyridyl, 2-chloroquinoline, acridine, phenanthridine, benzoquinoline, benzisoquinoline, naphthyridine, pyrazine, 4,6-dimethylpyrazine, 2,6-dimethylpyrazine, pyridazine, pyrimidine, quinoxaline, 2,3-dimethylquinoxaline, quinazoline, phthalazine, phenazine, cinnoline, pteridine and the like. These compounds may also be used in the form of a polymer such as polyvinylpyridine. The tertiary amine may be used either by adding it to a reactor separately from the reactants and other catalyst components or by previously treating it with a part of at least one catalyst component to form a suitable compound such as a complex or addition product thereof and thereafter adding this compound to the reactor. By way of example, palladium chloride, ferrous chloride and pyridine may be used either by adding them to the reactor separately from one another or by preparing a palladium chloridepyridine complex in advance and adding this complex, together with the ferrous chloride, to the reactor. They may also be used by preparing a ferrous chloridepyridine complex in advance and adding this complex, together with the palladium chloride, to the reactor. Furthermore, they may be used in the form of a mixture of complexes containing all of the palladium chloride, ferrous chloride and pyridine.

Such complexes can be prepared according to any suitable procedure for the preparation of conventional complexes. For example, a complex can be readily formed by agitating its components in a suitable solvent (such as benzene, monochlorobenzene, dichlorobenzene, ethanol, etc.) or an excess of a nitrogen-containing heterocyclic aromatic compound. A more active complex may be obtained if its preparation is carried out in an atmosphere of carbon monoxide. If necessary, the solvent or the excess of nitrogen-containing heterocyclic aromatic compound is distilled off.

The amine is preferably used in an amount of 0.3 to 5 moles per mole of the anion of the Lewis acid. It is especially suitable to use the amine in an amount equimolar with the anion of the Lewis acid. If the amount of amine used is less than 0.3 mole, its effect is unsatisfactory. On the other hand, it is possible to use more than 5 moles of the amine. In fact, the corrosion-inhibiting effect increases with the amount of amine used, but the reaction rate and yield show no significant improvement. Generally, it is unnecessary to use such a large excess of the amine.

Addition of the amine permits most of the catalyst to be recovered. Although the identity of the recovered catalyst is not clear, it can be recycled either directly or after being purified by a suitable technique such as solvent cleaning.

Where a nitrogen-containing heterocyclic aromatic compound as described above, together with a Lewis acid, is used as a co-catalyst, the reaction rate can further be enhanced by adding a small amount of water. The water is added in an amount of 1 to 70 moles and preferably 10 to 50 moles per mole of the aromatic nitro compound used as a raw material. If the amount of water added is less than 1 mole, practically no effect is produced, while if it is greater than 70 moles, the yield of the desired product is reduced greatly.

The iron hydroxide which is allowed to coexist in the reaction system according to the process of

the present invention can be ferrous hydroxide or ferric hydroxide. These compounds may be used alone or in admixture. Although no particular limitation is placed on the amount of iron hydroxide used, the iron hydroxide is generally used in such an amount that the weight ratio of iron hydroxide to aromatic nitro compound is in the range of 1 to $5 \times 10^{-2}$.

Although the process of the present invention can be carried out in the absence of solvent, any suitable solvent may be used. The solvents useful for this purpose include, for example, aromatic solvents such as benzene, toluene, xylene, etc.; nitriles such as acetonitrile, benzonitrile, etc.; sulfolane solvents such as sulfolane, etc.; halogenated aliphatic hydrocarbons such as 1,1,2-trichloro-1,2,2-trifluoroethane, etc.; halogenated aromatic hydrocarbons such as monochlorobenzene, dichlorobenzene, trichlorobenzene, etc.; ketones; esters; tetrahydrofuran; 1,4-dioxane; 1,2-dimethoxyethane; and the like.

In carrying out the process of the present invention, no particular limitation is placed on the manner in which the raw materials and the catalyst components are charged to a reactor. However, the nitro compound and the Lewis acid are desirably added after being totally or partially dissolved in the hydroxyl-containing compound or a suitable solvent.

Similarly, no particular limitation is placed on the order of addition, though it is restricted by the type of apparatus used. By way of example, a suitable pressure reactor such as autoclave is charged with a hydroxyl-containing compound, a catalyst, an aromatic nitro compound and an iron hydroxide. Then, carbon monoxide is introduced under pressure. The resulting mixture is heated and stirred until the desired reaction is completed. It is also possible to eliminate the carbon dioxide formed in the course of the reaction and feed carbon monoxide intermittently or continuously. The reaction may be carried out in batchwise, semicontinuous or continuous operation. The excess carbon monoxide remaining after completion of the reaction can be recycled to the process.

The reaction temperature is generally maintained in the range of 80° to 230°C and preferably 120° to 190°C. The reaction proceeds more rapidly as the temperature is raised, but reaction temperatures in the range of 130° to 170°C will suffice for ordinary purposes.

The reaction pressure, which is defined as the partial pressure of carbon monoxide, is in the range of 10 to 1000 kg/cm$^2$ and preferably 30 to 500 kg/cm$^2$. It is one of the important effects produced by the process of the present invention that the reaction time can be shortened by adding an iron hydroxide to the reaction system.

The reaction time may vary according to the nature of nitro compound used, reaction temperature, reaction pressure, type and amount of catalyst used, amount of iron hydroxide added, type of apparatus used, and the like. However, the reaction is generally carried out for a period of time ranging from 5 minutes to 6 hours. After completion of the reaction, the reaction mixture is cooled and the reactor is evacuated. Then, the reaction product is worked up according to a conventional procedure including filtration, distillation and other separation techniques, whereby the desired urethane is isolated from the unreacted materials, by-products, solvent, catalyst and the like.

The urethanes prepared by the process of the present invention have many uses as raw materials for the manufacture of agricultural chemicals, isocyanates and polyurethanes.

The present invention is further illustrated by the following examples. In all of these examples, the reaction was carried out by means of a magnetic stirring type autoclave made of stainless steel (SUS 32). The degrees of conversion and yields set forth in the examples were calculated from the results of analysis by gas chromatography and liquid chromatography.

### Example 1

Into an autoclave having a capacity of 200 ml were charged 10.92 g of 2,4-dinitrotoluene, 60 ml of absolute ethanol, 0.0038 g of palladium chloride, 1.64 g of a ferrous chloride-pyridine complex [formed by agitating a mixture (in a molar ratio of 1:2) of ferrous chloride and pyridine in ethanol], 0.21 g of water, and 1.2 of ferric hydroxide. The resulting mixture was allowed to react at an initial pressure of 90 kg/cm$^2$ and a temperature of 165°C. The completion of the reaction was confirmed by the fact that, 130 minutes after the temperature was raised, the pressure ceased to drop. Analysis of the discharged reaction mixture revealed that 2,4-dinitrotoluene was undetectable and the yield of diethyl toluene-2,4-dicarbamate (hereinafter referred to as diurethane) was 94.3%.

### Comparative Example 1

Reaction was carried out in the same manner as in Example 1, except that 0.0076 g of palladium chloride was used and the addition of ferric hydroxide was omitted. The absorption of carbon monoxide ceased in 150 minutes. Analysis of the reaction mixture revealed that the yield of diurethane was 92%. When the amount of palladium chloride was reduced to 0.0038 g, the time required for the reaction was as long as 360 minutes.

### Example 2

Reaction was carried out in the same manner as in Example 1, except that 0.045 g of 5% palladium-carbon was used in place of the palladium chloride (0.0038 g). The completion of the reaction was confirmed by the fact that, 180 minutes after the temperature was raised, the pressure

ceased to drop. Analysis of the discharged reaction mixture revealed that the yield of diurethane was 93%.

Example 3

Into an autoclave having a capacity of 200 ml were charged 10.92 g of 2,4-dinitrotoluene, 60 ml of isobutanol, 0.0076 g of palladium chloride, 0.82 g of a ferrous chloride-pyridine complex, 0.31 g of water, and 1.23 g of ferric hydroxide. The resulting mixture was allowed to react at an initial pressure of 90 kg/cm$^2$ and a temperature of 165°C. It was recognized that, 110 minutes after the temperature was raised, the pressure ceased to drop. Analysis of the discharged reaction mixture revealed that the yield of diurethane was 94%.

Comparative Example 2

Reaction was carried out in the same manner as in Example 3, except that the addition of 1.23 g of ferric hydroxide was omitted. The absorption of carbon monoxide ceased in 290 minutes. Analysis of the reaction mixture revealed that the yield of diurethane was 92%.

Example 4

Reaction was carried out in the same manner as in Example 3, except that 0.51 g of anhydrous ferric chloride and 0.99 g of isoquinoline were used in place of the ferrous chloride-pyridine complex (0.82 g). Analysis of the discharged reaction mixture revealed that the yield of diurethane was 93%.

Example 5

Into an autoclave having a capacity of 500 ml were charged 69 g of nitrobenzene, 140 ml of methanol, 17 mg of palladium chloride, 4 g of a ferrous chloride-pyridine complex, 3.2 g of water, and 3 g of ferric hydroxide. The resulting mixture was allowed to react at an initial pressure of 90 kg/cm$^2$ and a temperature of 160°C. It was recognized that, 50 minutes after the temperature was raised, the pressure ceased to drop. Analysis of the reaction mixture revealed that nitrobenzene was undetectable and the yield of phenylurethane was 97%.

Comparative Example 3

Reaction was carried out in the same manner as in Example 5, except that the addition of ferric hydroxide was omitted. The time required for the reaction was an long as 130 minutes, and the yield of phenylurethane was 97%.

**Claims**

1. A process for the preparation of aromatic urethanes by reacting an aromatic nitro compound, a hydroxyl-containing organic compound having at least one hydroxyl group, and carbon monoxide at high temperature and pressure in the presence of a catalyst composed of (a) a platinum group metal and/or a compound thereof, (b) a Lewis acid and (c) a tertiary amine, characterized in that an iron hydroxide is allowed to coexist in the reaction system.

2. A process as claimed in claim 1 wherein the aromatic nitro compound is an aromatic mono- or dinitro compound selected from the group consisting of nitrobenzene, *o*-, *m*- and *p*-chloronitrobenzenes, 2,3- and 3,4-dichloronitrobenzenes, *m*- and *p*-dinitrobenzenes, 2,4- and 2,6-dinitrotoluenes, and 1,5-dinitronaphthalene.

3. A process as claimed in claim 1 wherein the aromatic nitro compound is a substituted or unsubstituted nitrobenzene.

4. A process as claimed in claim 1 wherein the aromatic nitro compound is a substituted or unsubstituted dinitrotoluene.

5. A process as claimed in claim 1 wherein the hydroxyl-containing organic compound is methanol, ethanol or isobutanol.

6. A process as claimed in claim 1 wherein the platinum group metal and/or a compound thereof is palladium, a palladium compound, or a compound including a palladium compound.

7. A process as claimed in claim 1 wherein the Lewis acid is ferrous chloride or ferric chloride.

8. A process as claimed in claim 1 wherein the tertiary amine is a nitrogen-containing heterocyclic aromatic compound.

9. A process as claimed in claim 1 wherein the Lewis acid and the tertiary amine comprise a complex previously formed from ferrous chloride or ferric chloride and a nitrogen-containing heterocyclic aromatic compound.

10. A process as claimed in claim 1 wherein the reaction temperature is in the range of 80° to 230°C.

11. A process as claimed in claim 1 wherein the reaction pressure is in the range of 10 to 1000 kg/cm$^2$.

12. A process as claimed in claim 6 wherein the palladium compound is palladium chloride or palladium bromide.

13. A process as claimed in claim 6 wherein the palladium is supported on a carbon or alumina carrier.

14. A process as claimed in claim 8 or 9 wherein the nitrogen-containing heterocyclic aromatic compound is pyridine, quinoline or isoquinoline.

15. A process as claimed in claim 1 wherein the iron hydroxide is ferrous hydroxide.

16. A process as claimed in claim 1 wherein the iron hydroxide is ferric hydroxide.

17. A process as claimed in claim 1 wherein the iron hydroxide is used in such an amount that the weight ratio of iron hydroxide to aromatic nitro compound is in the range of 1 to $5 \times 10^{-2}$.

## Revendications

1. Procédé pour la préparation d'uréthannes aromatiques par réaction d'un composé nitro aromatique, d'un composé organique hydroxylé ayant au moins un groupe hydroxy, et de monoxyde de carbone, à température et à pression élevées, en présence d'un catalyseur composé de (a) un métal du groupe du platine et/ou un composé de ce métal, (b) un acide de Lewis et (c) une amine tertiaire, caractérisé en ce qu'on laisse un hydroxyde de fer coexister dans le système réactionnel.

2. Procédé selon la revendication 1, dans lequel le composé nitro aromatique est un composé mono- ou dinitro aromatique choisi parmi le groupe constitué par le nitrobenzène, les o-, m- et p-chloronitrobenzènes, les 2,3- et 3,4-dichloronitrobenzènes, les m- et p-dinitrobenzènes, les 2,4- et 2,6-dinitrotoluènes et le 1,5-dinitronaphtalène.

3. Procédé selon la revendication 1, dans lequel le composé nitro aromatique est un nitrobenzène substitué ou non substitué.

4. Procédé selon la revendication 1, dans lequel le compose nitro aromatique est un dinitrotoluène substitué ou non substitué.

5. Procédé selon la revendication 1, dans lequel le composé organique hydroxylé est le méthanol, l'éthanol, ou l'isobutanol.

6. Procédé selon la revendication 1, dans lequel le métal de groupe de platine et/ou un composé de ce métal est le palladium, un composé de palladium ou un composé comprenant un composé de palladium.

7. Procédé selon la revendication 1, dans lequel l'acide de Lewis est le chlorure ferreux ou le chlorure ferrique.

8. Procédé selon la revendication 1, dans lequel l'amine tertiaire est un composé hétérocyclique aromatique azoté.

9. Procédé selon la revendication 1, dans lequel l'acide de Lewis et l'amine tertiaire comprennent un complexe précédemment formé à partir de chlorure ferreux ou de chlorure ferrique et d'un composé hétérocyclique aromatique azoté.

10. Procédé selon la revendication 1, dans lequel la température de réaction est comprise dans la gamme de 80 à 230°C.

11. Procédé selon la revendication 1, dans lequel la pression de réaction est comprise dans la gamme de 9,81 à 981 bars (10 à 1000 kg/cm²).

12. Procédé selon la revendication 6, dans lequel le composé de palladium est le chlorure de palladium ou le bromure de palladium.

13. Procédé selon la revendication 6, dans lequel le palladium est fixé sur un support de carbone ou d'alumine.

14. Procédé selon la revendication 8 ou 9, dans lequel le composé hétérocyclique aromatique azoté est la pyridine, la quinoléine ou l'isoquinoléine.

15. Procédé selon la revendication 1, dans lequel l'hydroxyde de fer est l'hydroxyde ferreux.

16. Procédé selon la revendication 1, dans lequel l'hydroxyde de fer est l'hydroxyde ferrique.

17. Procédé selon la revendication 1, dans lequel l'hydroxyde de fer est utilisé en une quantité telle que le rapport pondéral de l'hydroxyde de fer au composé nitro aromatique est compris dans la gamme de 1 à $5 \times 10^{-2}$.

## Patentansprüche

1. Verfahren zur Herstellung aromatischer Urethane durch Reaktion einer aromatischen Nitro-Verbindung, einer hydroxyl-haltigen organischen Verbindung mit mindestens einer Hydroxyl-Gruppe und Kohlenstoffmonoxid bei hoher Temperatur und nohem Druck in Gegenwart eines Katalysators, der zusammengesetzt ist aus (a) einem Metall der Platin-Gruppe und/oder einer Verbindung desselben, (b) einer Lewis-Säure und (c) einem tertiären Amin, dadurch gekennzeichnet, daß man ermöglicht, daß ein Eisenhydroxid in dem Reaktionssystem koexistiert.

2. Verfahren nach Anspruch 1, dadurch gekennzeichnet, daß die aromatische Nitro-Verbindung eine aromatische Mono- oder Dinitro-Verbindung ausgewählt aus der aus Nitrobenzol, o-, m- und p-Chloronitrobenzol, 2,3- und 3,4-Dichloronitrobenzol, m- und p-Dinitrobenzol, 2,4- und 2,6-Dinitrotoluol und 1,5-Dinitronaphthalin bestehenden Gruppe ist.

3. Verfahren nach Anspruch 1, dadurch gekennzeichnet, daß die aromatische Nitro-Verbindung ein substituiertes oder unsubstituiertes Nitrobenzol ist.

4. Verfahren nach Anspruch 1, dadurch gekennzeichnet, daß die aromatische Nitro-Verbindung ein substituiertes oder unsubstituiertes Dinitrotoluol ist.

5. Verfahren nach Anspruch 1, dadurch gekennzeichnet, daß die hydroxyl-haltige organische Verbindung Methanol, Ethanol oder Isobutanol ist.

6. Verfahren nach Anspruch 1, dadurch gekennzeichnet, daß das das Metall der Platin-Gruppe und/oder die Verbindung desselben Palladium, eine Palladium-Verbindung oder eine eine Palladium-Verbindung einschließende Verbindung ist.

7. Verfahren nach Anspruch 1, dadurch gekennzeichnet, daß die Lewis-Säure Eisen(II)-chlorid oder Eisen(III)-chlorid ist.

8. Verfahren nach Anspruch 1, dadurch gekennzeichnet, daß das tertiäre Amin eine stickstoffhaltige heterocyclische aromatische Verbindung ist.

9. Verfahren nach Anspruch 1, dadurch gekennzeichnet, daß die Lewis-Säure und das tertiäre Amin einen Komplex umfassen, der aus Eisen(II)-chlorid oder Eisen(III)-chlorid und einer stickstoffhaltigen heterocyclischen aromatischen Verbindung vorher gebildet wurde.

10. Verfahren nach Anspruch 1, dadurch gekennzeichnet, daß die Reaktionstemperatur im Bereich von 80°C bis 230°C liegt.

11. Verfahren nach Anspruch 1, dadurch gekennzeichnet, daß der Reaktionsdruck im Bereich von 9,81 bis 981 bar (10 bis 1000 $kg/cm^2$) liegt.

12. Verfahren nach Anspruch 6, dadurch gekennzeichnet, daß die Palladium-Verbindung Palladiumchlorid oder Palladiumbromid ist.

13. Verfahren nach Anspruch 6, dadurch gekennzeichnet, daß das Palladium auf einen Kohlenstoff- oder Aluminiumoxid-Träger aufgezogen ist.

14. Verfahren nach Anspruch 8 oder 9, dadurch gekennzeichnet, daß die stickstoffhaltige heterocyclische aromatische Verbindung Pyridin, Chinolin oder Isochinolin ist.

15. Verfahren nach Anspruch 1, dadurch gekennzeichnet, daß das Eisenhydroxid Eisen(II)-hydroxid ist.

16. Verfahren nach Anspruch 1, dadurch gekennzeichnet, daß das Eisenhydroxid Eisen(III)-hydroxid ist.

17. Verfahren nach Anspruch 1, dadurch gekennzeichnet, daß das Eisenhydroxid in einer solchen Menge verwendet wird, daß das Gewichtsverhältnis des Eisenhydroxids zu der aromatischen Nitro-Verbindung im Bereich von 1 bis $5 \times 10^{-2}$ liegt.